Europäisches Patentamt

European Patent Office

Office européen des brevets

(19)

(11) Veröffentlichungsnummer: **0 177 685**
**A2**

(12) # EUROPÄISCHE PATENTANMELDUNG

(21) Anmeldenummer: **85108827.8**

(51) Int. Cl.⁴: **A 61 L 2/04**

(22) Anmeldetag: **15.07.85**

(30) Priorität: **20.09.84 DE 3434472**

(43) Veröffentlichungstag der Anmeldung: **16.04.86**
Patentblatt 86/16

(84) Benannte Vertragsstaaten: **AT BE CH DE FR GB IT LI LU NL SE**

(71) Anmelder: **SCHWAB & CO. GES.M.B.H., Doerenkampgasse 4, A-1100 Wien (AT)**

(84) Benannte Vertragsstaaten: **BE CH FR GB IT LI LU NL SE AT**

(71) Anmelder: **Lentia Gesellschaft mit beschränkter Haftung, Arabellastrasse 4 Postfach 81 05 08, D-8000 München 81 (DE)**

(84) Benannte Vertragsstaaten: **DE**

(72) Erfinder: **Doleschel, Walter, Prof. Dr., Sonnleitensteig 7, A-1190 Wien (AT)**
Erfinder: **Kaltschmid, Helmut, Dr., Dammgasse 11, A-2540 Bad Vöslau (AT)**

(74) Vertreter: **Kunz, Ekkehard, Chemie Linz AG Patentabteilung St. Peter-Strasse 25, A-4020 Linz (AT)**

(54) **Verfahren zur Pasteurisation von Plasmaproteinen bzw. von Plasmaproteinfraktionen.**

(57) Pasteurisation von pulverförmigen, lyophilisierten Plasmaproteinen in Suspension in einem bei Pasteurisationstemperatur flüssigen und bei dieser Temperatur nicht zersetzlichen organischen Wärmeüberträger, der auf die Plasmaproteine nur «in geringem Ausmaß denaturierend» wirkt. Dieser Wärmeüberträger wird bevorzugt aus der Gruppe der aliphatischen, cycloaliphatischen und aromatischen Kohlenwasserstoffe, der aliphatischen Monoalkohole mit mindestens 3 C-Atomen und der öligen Substanzen ausgewählt.

- 1 -

Verfahren zur Pasteurisation von Plasmaproteinen bzw. von Plasmaproteinfraktionen.

Die vorliegende Erfindung betrifft ein Verfahren zur Pasteurisation von
Plasmaproteinen bzw. von Fraktionen derselben, bei dem nur eine sehr
begrenzte Denaturierung der Plasmaproteine in Kauf genommen werden muß.

Die Behandlung von Patienten mit Proteinen oder Proteinfraktionen, die aus
humanen Blutplasma hergestellt wurden, birgt immer das Risiko der Übertragung von Krankheiten, insbesondere von Hepatitis in sich, wenn auch dieses
Risiko durch sorgfältige Spenderausahl und eingehende Testung gering gehalten
werden kann. Bei der laufenden Gabe von Plasmaproteinen, wie sie z. B. bei
Patienten, die an Haemophilie leiden, nötig ist, fällt das Infektionsrisiko
allerdings mehr ins Gewicht.

Seit einiger Zeit sind Bestrebungen im Gange, dieses Infektionsrisiko durch
eine Hitzebehandlung dieser Plasmaproteine oder einzelner seiner Fraktionen
auszuschalten. Dem steht entgegen, daß sich zwar Albumin und hitzestabile
alpha und beta Globuline unter gewissen Vorkehrungen in wäßriger Lösung
pasteurisieren lassen, nicht aber andere Fraktionen wie z. B. Immunglobuline,
Gerinnungsfaktoren, vor allem Faktor VIII Konzentrate. Diese werden bei der
Hitzebehandlung praktisch vollständig denaturiert.

Es wurden zwar verschiedene stabilisierende Zusätze wie Saccharide, Zuckeralkohole, Aminosäuren, Ca-Ionen, K- oder Ammoniumcitrat, Salze von Carbonsäuren bzw. Hydroxycarbonsäuren vorgeschlagen (EP-A 18.561, EP-A 35.204, EP-B 37.078, EP-A 77.870, EP-A 106.269, DE-A 33 30 770 sowie PCT-Anmeldung WO 83/04027), doch sind hier die Denaturierungsraten immer noch ziemlich hoch.

Aus der EP-A 0 094 611 bzw. der EP-A 0 110 407 ist bekannt, daß sich trockene, z. B. lyophilisierte Plasmaproteine, besonders solche die Faktor VIII enthalten, in trockenem Zustand mit geringerem Verlust an Aktivität pasteurisieren lassen. So müssen laut diesen Europäischen Patentanmeldungen bei Pasteurisationstemperaturen von 65 - 75°C nur 20 - 10 % Aktivitätsverlust bei Faktor VIII Präparaten in Kauf genommen werden. Derartige Trockenerhitzungen sind aber, vor allem bei größeren Mengen, sehr schwierig durchzuführen, da die Wärmeübertragung in solchen Präparaten sehr schlecht und ungleichmäßig ist, was einerseits partielle Überhitzungen und andererseits partielle ungenügende Erhitzung zur Folge hat. Es ist auch die völlige Virusinaktivierung dabei nicht immer gewährleistet.

Gemäß EP 0 112.563 wurde schließlich vorgeschlagen, eine Beseitigung von infektiösen Viren, insbesondere Hepatitis B- sowie non-A-non-B-Virus sowie von anderen pyrogenen Substanzen mit Lipidstruktur durch eine extraktive Behandlung mit fettlösenden Lösungsmitteln wie Chloroform, Aceton, Diäthyläther, Benzol, Toluol, Xylol, Methanol, Äthanol, Isopropanol, n-Octanol, Tetrahydrofuran oder Mischungen derselben bei etwa 0°C bis 20°C, vorzugsweise 2°C bis 10°C durchzuführen, wobei diese Behandlung etwa 1 - 8 Stunden dauern soll. Die Serumproteine sollen dabei 50 - 90 % ihrer ursprünglichen Aktivität beibehalten, es wird jedoch ausdrücklich darauf hingewiesen, daß Temperaturen über 30°C wegen der dann weiter zunehmenden Schädigung der Plasmaproteine auszuschließen sind. Dieses Verfahren hat den Nachteil, daß nur jene infektiös wirkenden Substanzen damit beseitigt werden, die Lipidstruktur besitzen, solche anderer Struktur jedoch unangegriffen bleiben.

Überraschenderweise konnte nun gefunden werden, daß eine wirksame und überaus schonende Pasteurisation von Plasmaproteinen dann möglich ist, wenn die Plasmaproteine als Suspension in einem bei Pasteurisationstemperatur flüssigen und stabilen organischen Wärmeüberträger der Pasteurisation unterworfen werden, wobei der verwendete Wärmeüberträger unter weiter unten genannten Bedingungen als "in geringem Ausmaß denaturierend" auf Plasmaproteine wirkend festgestellt worden sein muß. Bei dieser schonenden Pasteurisierung ist eine gleichmäßige Wärmeverteilung gewährleistet und daher eine schädliche lokale Überhitzung vermieden, wobei Aktivitätsverluste auch bei Faktor VIII Konzentraten, von unter 10 % unschwer erzielbar sind.

Gegenstand der vorliegenden Erfindung ist demnach ein Verfahren zur Pasteurisation von Plasmaproteinen bzw. von Plasmaproteinfraktionen, das dadurch gekennzeichnet ist, daß man die Pasteurisation an pulverförmigen, lyophilisierten Plasmaproteinen oder Plasmaproteinfraktionen durchführt, die in Suspension in einem bei Pasteurisationstemperatur flüssigen und bei dieser Temperatur nicht zersetzlichen organischen Wärmeüberträger, in dem die Plasmaproteine nicht löslich sind und der auf die Plasmaproteine nur "in geringem Ausmaß denaturierend" wirkt, oder in mehreren solcher Wärmeüberträger, die miteinander mischbar sind, vorliegen.

Der vorliegenden Erfindung liegt die überraschende Erkenntnis zugrunde, daß nicht alle organischen Lösungsmittel oder sonstigen inerten, bei Pasteurisationstemperatur flüssigen Substanzen die in der EP-A 0 112.563 aufgezeigte, starke denaturierende Wirkung auf Plasmaproteine besitzen, wenn Temperaturen von 30°C oder höher angewandt werden. Es gibt vielmehr eine Reihe von organischen Lösungsmitteln, die ein Erwärmen auf die üblichen Pasteurisationstemperaturen gestatten, ohne daß darin suspendierte Plasmaproteine in nennenswertem Ausmaß geschädigt werden. Unter auf Plasmaproteine nur "in geringem Ausmaß denaturierend" wirkende Lösungsmittel sind im Sinne der vorliegenden Erfindung solche zu verstehen, mit denen ein darin suspendiertes Faktor VIII Konzentrat nach 2-stündigem Erhitzen auf 60°C mehr als 75 % seiner biologischen Aktivität beibehält. Dieser Auswahltest wird wie folgt durchgeführt:

1 g lyophilisiertes AHF-Pulver mit einer spezifischen Aktivität von Faktor VIII von etwa 0,7 Einheiten/mg Protein, wird mit 100 ml des zu prüfenden Wärmeüberträgers im Rotationskolben an einem Rotationsverdampfer oder in einem Rührwerksbehälter mit Rückflußkühler mittels Wasserbad unter Drehen bzw. Rühren 2 Stunden auf 60°C gehalten. Das suspendierte Protein wird anschließend abfiltriert oder abgenutscht und zweimal mit je etwa 50 ml an einem leicht flüchtigen Lösungsmittel gewaschen, das mit dem verwendeten Wärmeüberträger mischbar ist. Nach Luft- und Vakuumtrocknung wird im so erhaltenen Präparat eine Faktor VIII Bestimmung nach Simone, van der Heiden, Abilgaard, J. Lab-Clin. Med. 69, 706, 1967 durchgeführt.

Lösungsmittel, die den Anforderungen dieses Tests genügen, sind vorzugsweise unter den aliphatischen cycloaliphatischen oder aromatischen Kohlenwasserstoffen, unter aliphatischen Monoalkoholen mit mehr als 2 C-Atomen, insbesondere unter den geradkettigen Monoalkoholen, sowie unter öligen Substanzen wie z. B. Silikonölen zu finden. Verzweigte Alkohole wirken meist stärker denaturierend als die geradkettigen Isomeren.

Säuren erfüllen die Bedingungen des Tests meist nicht. Ebenso ungeeignet sind stark polare Lösungsmittel wie Dimethylformamid und Acetonitril. Ebenfalls zu stark denaturierend im Sinne der vorliegenden Erfindung wirkt Chloroform.

In der Folge werden Denaturierungswerte für einige repräsentative Lösungsmittel, die nach der oben angegebenen Methode überprüft wurden, und die der Erfordernis eines Erhaltes von mindestens 75 % der Aktivität entsprochen haben, angegeben.

## Tabelle I

| Wärmeüberträger | Aktivität nach 2-stündiger Behandlung bei 60°C in % des Ausgangswertes |
| --- | --- |
| Cyclohexan | 99,1 |
| n-Heptan | 99,5 |
| Benzol | 99,2 |

| Paraffinöl | 99,7 |
| n-Propanol | 98,0 |
| n-Butanol (Wassergehalt rund 0,4 %) | 88,1 |
| n-Pentanol (Wassergehalt rund 0,4 %) | 78,0 |
| n-Octanol | 81,7 |
| Isopropanol | 96,0 |
| 2-Butanol | 97,8 |
| Isobutanol | 93,0 |
| tert.Butanol | 91,7 |

Ähnlich gute Ergebnisse werden mit Lösungsmitteln, wie Toluol, Xylol, Butylacetat, oder Äthylenglykolmonomethyläther erzielt.

Hingegen ergaben z. B. folgende andere Lösungsmittel Aktivitätswerte nach erfolgter Behandlung von unter 75 % der Ausgangsaktivität und wurden daher als nicht geeignet eingestuft.

Tabelle II

| Wärmeüberträger | Aktivität nach 2-stündiger Behandlung bei 60°C in % des Ausgangswertes |
| --- | --- |
| Methanol | vollständig denaturiert |
| Äthanol | 69,8 |
| Tetrahydrofuran | 71,2 |
| Methyläthylketon | 71,0 |
| Essigsäureäthylester | 73,7 |
| Acetonitril | 67,7 |
| Dimethylformamid | 46,4 |
| Hexansäure | vollständig denaturiert |
| Chloroform | 46,5 |
| Äthylenglykol | vollständig denaturiert |

Lösungsmittel, die als Wärmeüberträger für das erfindungsgemäße Verfahren vorgesehen sind, sollen möglichst wasserfrei sein. Schon geringe Wassermengen im System können zu stärkeren Denaturierungen führen. Es ist daher zweckmäßig, Wassergehalte im System von über 1 Gew.% zu vermeiden. So wird z.B. bei tert.Butanol durch die Gegenwart von etwa 2 % Wasser die denaturierende Wirkung so erhöht, daß die Kennzahl des Tests nur mehr 65,9 % beträgt. Verwendet man Wärmeüberträger, die mit Wasser nicht mischbar sind, so ist dem Wassergehalt des Systems mehr Augenmerk zu schenken, als in jenen Fällen, in denen der Wärmeüberträger mit Wasser mischbar ist und vermutlich so der direkten Einwirkung auf die Plasmaproteine mehr entzogen ist. So wird z. B. die denaturirende Wirkung von n-Heptan durch die Gegenwart von nur 0,2 % Wasser im System so erhöht, daß im oben angeführten Test nur mehr 63,3 % der ursprünglichen Aktivität erhalten bleibt, während bei 0,2 % Wasser im System und n-Propanol als Wärmeüberträger immerhin 83,8 % der ursprünglichen Aktivität festgestellt wurden und auch n-Butanol und n-Pentanol mit einem Wassergehalt von etwa 0,4 % die Erfordernisse des Tests noch erfüllen, wie Tabelle 1 zeigt.

Im Allgemeinen sollte daher zweckmäßig dafür gesorgt werden, daß bei Verwendung von Wärmeüberträgern, die mit Wasser mischbar sind, ein Wassergehalt von 0,5 Gew.% nicht überschritten wird, während bei mit Wasser nicht mischbaren Lösungsmitteln die Einhaltung eines maximalen Wassergehalts von 0,2 % empfehlenswert ist. Diese empfohlenen Grenzen für tolerierbare Wasserwerte im System sind jedoch nur Richtwerte, da Schwankungen abhängig vom Lösungsmittel und von der eingesetzten Serumproteinfraktion bestehen. In allen Fällen empfiehlt es sich daher, vor Einsatz eines Lösungsmittels dessen Brauchbarkeit im oben angegebenen Test zu überprüfen, um unerwünschte Fehlschläge zu vermeiden.

Ist ein verwendeter Wärmeüberträger bei der gewählten Pasteurisationstemperatur bereits flüchtig, sollte zweckmäßig durch geeignete Maßnahmen, z. B. durch Anwendung eines Rückflußkühlers dafür gesorgt werden, daß das Volumen während der Pasteurisierung etwa konstant bleibt.

Die erfindungsgemäß ausgewählten Wärmeüberträger können einzeln oder auch als Mischungen untereinander, sofern völlige Mischbarkeit vorliegt, eingesetzt werden.

Temperatur und Behandlungsdauer der erfindungsgemäßen Pasteurisation richten sich nach den für die Pasteurisation von Blutfraktionen gebräuchlichen Werten. Natürlich ist auch dem Siedepunkt des Wärmeüberträgers Rechnung zu tragen. Im allgemeinen ist eine Pasteurisation bei 55 bis 65°C bei einer Dauer von 8 - 12 Stunden zu empfehlen, da hier der Vorteil von geringer Denaturierung und wirksamer Zerstörung von infektiösen Substanzen miteinander verbunden werden können. Natürlich nimmt die denaturierende Wirkung der Behandlung mit der Dauer und der angewendeten Temperatur meist zu, sodaß mit einer höheren Denaturierungsrate zu rechnen ist, die aber im Vergleich zu den Verfahren gemäß dem Stand der Technik immer noch relativ günstig liegt. So wird z. B. bei einer Pasteurisation von 2 Stunden bei 98,4°C mit n-Heptan als Wärmeüberträger immer noch eine Aktivität von 53,2 % des Ausgangswertes verzeichnet.

Im allgemeinen können Temperaturen von 50 - 120°C und Pasteurisationszeiten von 2 - 20 Stunden Anwendung finden, es sind aber auch andere Varianten wie z. B. schockartige Erhitzung von nur wenigen Sekunden möglich. In solchen Fällen empfehlen sich vor allem hochsiedende Wärmeüberträger wie z. B. hochsiedende aromatische Kohlenwasserstoffe wie Toluol oder Xylol, Silikonöle oder Paraffinöle. Voraussetzung ist immer, daß eine wirksame Zerstörung der infektiösen Substanzen gewährleistet ist.

Die Aufarbeitung der Mischungen nach beendeter erfindungsgemäßer Pasteurisation gestaltet sich sehr einfach. Der verwendete Wärmeüberträger kann durch Abfiltrieren oder Abnutschen abgetrennt werden.

Ist der eingesetzte Wärmeüberträger schwer flüchtig oder gar hochsiedend, empfiehlt es sich, die zurückbleibenden Serumproteine mit einem leichtflüchtigen Lösungsmittel, das mit dem verwendeten Wärmeüberträger mischbar ist, einmal oder vorzugsweise mehrmals nachzuwaschen und dann das Präparat zu trocknen. Es kann dann auf jede übliche Weise für die Bereitung von Plasmaproteinpräparaten, z. B. unter Auflösen in Wasser, Mischen mit anderen

-8-

Zusätzen, Einstellen bestimmter Konzentrationen, weiterverarbeitet werden. Als Waschlösungsmittel hat sich in vielen Fällen n-Hexan wegen seiner guten Flüchtigkeit besonders bewährt.

Es sind aber auch andere flüchtige Lösungsmittel, z. B Diäthyläther, Aceton brauchbar.

Eine andere Möglichkeit der Aufarbeitung, die dann anwendbar ist, wenn ein mit Wasser mischbarer Wärmeüberträger eingesetzt wird, besteht darin, daß die Suspension nach beendeter Pasteurisation abgekühlt und solange mit Wasser verdünnt wird, bis das Plasmaprotein in Lösung gegangen ist. Aus der so erhaltenen, meist opaleszierenden Lösung kann das Plasmaprotein mit üblichen Proteinfällungsmitteln, wie z. B. Äthylalkohol, Polyäthylenglykol, Glycin, Aussalzmittel wie NaCl oder Ammonsulfat oder Kombinationen dieser Fällungsmittel ausgefällt und der üblichen Endfertigung zugeführt werden.

Mit dem erfindungsgemäßen Verfahren können lyophilisierte Plasmaproteine mit gutem Erfolg pasteurisiert werden. Insbesondere gilt dies für Frischplasma, Immunserumglobulin, sowohl für i.V.-als auch i.M-Verabreichung, Kryopräzipität, Cohn I Fraktion, Faktor VIII Konzentrat, Faktor IX Konzentrat, Prothrombinkomplex, Fibrinogen, Fibronectin und Antithrombin III, die alle in lyophilisiertem Zustand eingesetzt werden. Dabei wird gute Infektionssicherheit erzielt.

In den nachfolgenden Beispielen wird das erfindungsgemäße Verfahren näher erläutert:

Beispiel 1:

Lyophilisiertes, pulverförmiges Frischplasma wurde in n-Heptan als Wärmeüberträger suspendiert, wobei pro Gramm Frischplasma 100 g Wärmeüberträger eingesetzt wurden. Die Suspension wurde dann am Rotationsverdampfer 10 Stunden bei 60°C pasteurisiert. Nach beendeter Pasteurisation wurde der Niederschlag von n-Heptan abfiltriert, zweimal mit n-Hexan gewaschen und zuerst in Luft und anschließend im Vakuum getrocknet.

Die biologische Aktivität der einzelnen Fraktionen in diesem Frischplasma betrug die folgenden Prozentsätze vom Ausgangswert vor der Pasteurisation.

| | |
|---|---|
| Faktor VIII | 92,3 % |
| Faktor IX | 93,2 % |
| Faktor II | 89,0 % |
| Immunglobulin | |
| IgG | 97,0 % |
| IgA | 99,5 % |

Beispiel 2:

Lyophilisiertes i. V. Immunglobulin wurde wie in Beispiel 1 angegeben in n-Heptan 10 Stunden bei 60°C pasteurisiert und wie in Beispiel 1 aufgearbeitet. Das Immunglobulin zeigte nach der Pasteurisation folgende Kenndaten:

a) Bestimmung des Polymergehaltes durch Ultrazentrifugation

| | vor Pasteurisation | nach Pasteurisation |
|---|---|---|
| Polymere und Dimere | 8 rel % | 9,5 rel % |
| Monomere | 92 rel % | 90,5 rel % |

Es hat also keine nennenswerte Denaturierung stattgefunden.

b) Antikomplementäraktivität

| vor Pasteurisation | nach Pasteurisation |
|---|---|

-10-

% Komplement von 100 %   16 %                    12 %

Die Antikomplementäraktivität stieg also durch die Pasteurisation nicht nur nicht an, sondern wurde niedriger.

c) Radiale Immundiffusion

|       | vor Pasteurisation | nach Pasteurisation |
|-------|--------------------|---------------------|
| IgA   | 120 mg/dl          | 115 mg/dl           |
| IgG   | 4820 mg/dl         | 4560 mg/dl          |

Beispiel 3:

Lyophilisiertes i.m.Immunglobulin wurde wie in Beispiel 1 beschrieben 10 Stunden bei 60°C mit n-Heptan als Wärmeüberträger pasteurisiert, anschließend abfiltriert und wie in Beispiel 1 aufgearbeitet. Das pasteurisierte Immunglobulin zeigte folgende Werte:

Bestimmung des Polymergehaltes durch Ultrazentrifugation:

|          | vor Pasteurisation | nach Pasteurisation |
|----------|--------------------|---------------------|
| Polymere | 1,9 %              | 2,5 %               |
| Dimere   | 15,1 %             | 16,8 %              |
| Monomere | 83,0 %             | 80,7 %              |

Es fand also keine nennenswerte Aggregatbildung statt.

Beispiel 4:

Lyophilisierter Prothrombin-Komplex wurde wie in Beispiel 1 beschrieben mit n-Heptan als Wärmeüberträger 10 Stunden bei 60°C pasteurisiert und anschließend wie in Beispiel 1 beschrieben weiterbehandelt.
Die Aktivitätsausbeute betrug an Faktor IX 90,3 %, an Faktor II 93,0 % und an Faktor X 97,3 %. Es waren keine aktivierten Faktoren zu verzeichnen.

Beispiel 5:

Lyophilisiertes Fibrinogen wurde wie in Beispiel 1 beschrieben mit n-Heptan als Wärmeüberträger 10 Stunden bei 60°C pasteurisiert und wie in Beispiel 1 beschrieben aufgearbeitet.

Gerinnbares Protein vor Pasteurisation: 95 % des Gesamtproteins Gerinnbares Protein nach Pasteurisation: 93 % des Gesamtproteins

Untersuchung mit der Ultrazentrifuge:

| | Sedimentationskonstante | | | |
| | 7,6 S | 13,4 S | 18,6 S | 21,3 S |
| --- | --- | --- | --- | --- |
| vor<br>Pasteurisation<br>in rel % | 76,2 | 16,8 | 4,4 | 2,6 |
| nach | 77,0 | 15,8 | 4,7 | 2,5 |

Beispiel 6:

Lyophilisiertes AHF-Pulver mit einer spezifischen Aktivität von ungefähr 0,7 Einheiten Faktor VIII/mg Protein (enthaltend geringe Mengen an Citrat, Natriumchlorid und Glycin) wurde mit dem in der nachfolgenden Tabelle angegebenen Wärmeüberträger 10 Stunden bei 60°C pasteurisiert. Die Aufarbeitung erfolgte wie in Beispiel 1 beschrieben, wobei in allen Fällen n-Hexan als Waschflüssigkeit diente. Die Aktivitätsausbeute ist in nachfolgender Tabelle zusammengefaßt:

Tabelle III

| Wärmeüberträger | Aktivitätsausbeute % |
| --- | --- |
| Cyclohexan | 93,2 |
| n-Heptan | 93,0 |
| Benzol | 92,2 |
| Paraffinöl | 87,2 |
| Siliconöl | 87,2 |

-12-

Beispiel 7:

50 mg lyophiliertes Antithrombin III-Konzentrat-Pulver mit einer Antithrombin-III-Aktivität von 97 internationalen Einheiten wurden in 20 ml n-Heptan suspendiert und 10 Stunden bei 60°C pasteurisiert. Anschließend wurde der Feststoff abgenutscht, mit 10 ml n-Hexan ausgewaschen und im Vakuum getrocknet. Die Aktivitätsbestimmung des so erhaltenen pasteurisierten Antithrombin-III-Konzentrats erfolgte gemäß der Methode von J.Fareed, H.C. Messmore und J.U.Balis, Chromogenic Peptide Substrates, Seiten 183 -195, Churchill-Livingstone, Edinburg 1979, durchgeführt. Das pasteurisierte Produkt zeigte dabei 93,6 % der Ausgangsaktivität.

Beispiel 8:

0,5 g lyophilisiertes AHF-Pulver mit einer spezifischen Aktivität von Faktor VIII von etwa 0,7 Einheiten/mg Protein wurde in 40 ml Isobutanol suspendiert und insgesamt 16 Stunden unter Rühren in einem Rührwerksbehälter mit Rückflußkühler unter Beheizung mittels Wasserbad auf 60°C erhitzt, wobei in regelmäßigen Zeitabständen die Aktivitätsausbeute, ausgedrückt in Prozenten der Ausgangsaktivität, bestimmt wurde. Zur Aufarbeitung wurde das pasteurisierte Protein vom Wärmeüberträger durch Abnutschen abgetrennt, worauf es zweimal mit n-Hexan gewaschen wurde. Das Produkt wurde im Vakuum getrocknet. Die erhaltenen Aktivitätsausbeuten sind in nachfolgender Tabelle zusammengefaßt.

| Pasteurisationsdauer Stunden | Aktivitätsausbeute |
|---|---|
| 0 | 100 % |
| 2 | 93,0 % |
| 4 | 87,8 % |
| 8 | 81,7 % |
| 10 | 77,7 % |
| 16 | 69,5 % |

-13-

Patentansprüche:

1. Verfahren zur Pasteurisation von Plasmaproteinen bzw. von Plasmaproteinfraktionen, dadurch gekennzeichnet, daß man die Pasteurisation an pulverförmigen, lyophilisierten Plasmaproteinen oder Plasmaproteinfraktionen durchführt, die in Suspension in einem bei Pasteurisationstemperatur flüssigen und bei dieser Temperatur nicht zersetzlichen organischen Wärmeüberträger, in dem die Plasmaproteine nicht löslich sind, und der auf die Plasmaproteine nur "in geringem Ausmaß denaturierend" wirkt, oder in mehreren solcher Wärmeüberträger, die untereinander mischbar sind, vorliegen.

2. Verfahren nach Anspruch 1, dadurch gekennzeichnet, daß das Volumen der Plasmaproteinsuspension während der Pasteurisation konstant gehalten wird.

3. Verfahren nach den Ansprüchen 1 und 2, dadurch gekennzeichnet, daß der Wassergehalt im Pasteurisationssystem maximal 1 Gew.% beträgt.

4. Verfahren nach Anspruch 3, dadurch gekennzeichnet, daß ein mit Wasser nicht oder nur bis maximal 5 % mischbarer Wärmeüberträger verwendet wird und der Wassergehalt im System maximal 0,2 Gew.% beträgt.

5. Verfahren nach Anspruch 3, dadurch gekennzeichnet, daß der Wärmeüberträger mit Wasser mischbar ist und der Wassergehalt des Systems maximal 0,5 Gew.% beträgt.

6. Verfahren nach den Ansprüchen 1 bis 5, dadurch gekennzeichnet, daß das Plasmaprotein bzw. dessen Fraktion nach beendeter Wärmebehandlung abfiltriert, mit einem mit dem Wärmeüberträger mischbaren, leicht verdunstbaren Lösungsmittel ausgewaschen und anschließend schonend getrocknet wird.

7. Verfahren nach den Ansprüchen 1 bis 5, dadurch gekennzeichnet, daß bei Einsatz eines mit Wasser mischbaren Wärmeüberträgers die Suspension nach beendeter Pasteurisation abgekühlt, unter Rühren mit Wasser bis zur Erzielung einer Lösung des Plasmaproteins verdünnt und aus der Lösung das Plasmaprotein mit üblichen Proteinfällungsmitteln gefällt und abgetrennt wird.

8. Verfahren nach den Ansprüchen 1 bis 7, dadurch gekennzeichnet, daß 8 bis 12 Stunden bei einer Temperatur von 55 bis 65°C pasteurisiert wird.

9. Verfahren nach den Ansprüchen 1 bis 8, dadurch gekennzeichnet, daß als Wärmeüberträger ein aliphatischer, cycloaliphatischer oder aromatischer Kohlenwasserstoff eingesetzt wird.

10. Verfahren nach den Ansprüchen 1 bis 8, dadurch gekennzeichnet, daß als Wärmeüberträger ein aliphatischer Monoalkohol mit mindestens 3 C-Atomen eingesetzt wird.

11. Verfahren nach Anspruch 10, dadurch gekennzeichnet, daß der aliphatische Monoalkohol geradkettig ist.

12. Verfahren nach den Ansprüchen 1 bis 8, dadurch gekennzeichnet, daß als Wärmeüberträger ein Silikonöl eingesetzt wird.

O.Z. 776
4. 7.1985